# EUROPEAN PATENT APPLICATION

(11) **EP 1 980 281 A1**
(43) Date of publication of application: **15.10.2008**
(21) Application number: 07105923.2
(22) Date of filing: 11.04.2007
(51) Int. Cl.: A61M 1/36, A61M 25/00

(54) **Percutaneous access system**

(71) Applicant: Edwards Lifesciences Corporation, Irvine, CA 92614 (US)
(72) Inventor: Hoffman, Brent, Irvine, CA 92614-5688 (US); Mitchell, Sean, Irvine, CA 92614-5688 (US); Dummer, C Wade, Irvine, CA 92614-5688 (US)
(74) Representative: Parry, Christopher Stephen

(57) **Abstract**

A transition device (200) for placement at the opening of the tip of a cannula body (308) for the purpose of controlling the shape of the cannula tip opening under various loads such as might be encountered during device placement.

## Description

### BACKGROUND

### 1. Field of the Invention

This invention relates to the field of medical technology and medical devices and more particularly to the process of cannulation of the cardiovascular system to remove or return blood or other fluids through a cannula device.

### 2. Related Art

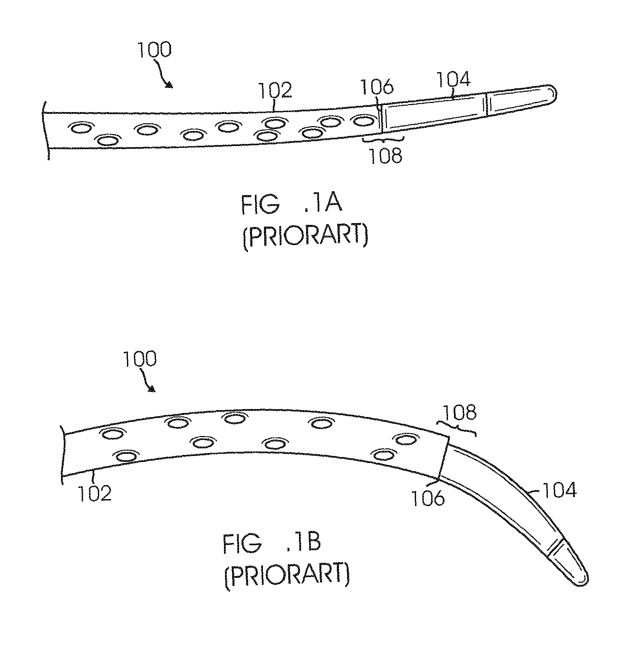

The process of cannulation of the cardiovascular system to remove or return blood or other fluids through a cannula device is known. One example, shown in FIG. 1A, is a venous cannulation system 100, including a cannula 102 and an introducer 104 designed to remove blood from the venous side of the circulatory system during a surgical procedure that requires cardiopulmonary bypass (CPB).

Cannula 102 should preferably be deflectable at its distal end 106 to permit proper positioning of distal end 106 of cannula 102 including introducer 104 within the femoral vein.

Frequently, the distal end or tip 106 of cannula 102 is made from a soft material, such as soft Polyurethane (PU) tubing, tipped to provide a smooth tapered profile for insertion into the femoral vein. Generally, insertion is done either through percutaneous access (Seldinger technique) or a standard cut-down access.

The soft PU tubing is prone to deformation or compression under resistance, for example when pushing through connective tissue such as might be encountered during a Seldinger technique access to the femoral vein. Such deformation causes difficulty for the surgeon by increasing the drag force on cannula 102 during insertion.

Additionally, as shown in FIG. 1B, the junction 108 between cannula 102 and introducer 104 is prone to exposing an edge during cannulation if introducer 104 is bent.

### SUMMARY

In light of the foregoing background, an improved device and associated method for the process of cannulation of the cardiovascular system are provided according to the various embodiments of the present invention.

This present invention relates to a transition device for placement at the opening of the tip of a cannula body for the purpose of controlling the shape of the cannula tip opening under various loads such as might be encountered during device placement.

In one aspect, a cannulation system is provided including a cannula body having a distal end and a first lumen extending therein. The system also includes an introducer configured to be insertably received into the distal end of the cannula body; and a transition device including a first section having an attachment geometry, a second section having a variable external diameter and an inner lumen extending therein. The first section is inserted into the first lumen at the distal end of the cannula body to secure the transition device thereto. The second section extends outside of the cannula body and is configured to allow the introducer into the first lumen of the cannula body. The second section provides a transition between the outer surface of the cannula body and the outer surface of the introducer such that no gap is opened at the intersection of the outer surface of the cannula body and the outer surface of the introducer as the introducer is flexed relative to the cannula body.

### BRIEF DESCRIPTION OF THE DRAWINGS

Having thus described the invention in general terms, reference will now be made to the accompanying drawings, which are not necessarily drawn to scale, and wherein:

FIGS. 1A and 1B are simplified views of a cannulation system;

FIG. 2A is a simplified perspective view of a transition device in accordance with an embodiment of the present invention;

FIG. 2B is a cross-sectional view of the transition device of FIG. 2A, including detailed sections, in accordance with an embodiment of the present invention;

FIG. 3 is a simplified cross-sectional view of a cannulation system including a transition device in accordance with an embodiment of the present invention;

FIG. 4A is a simplified perspective side view of a transition device in accordance with an embodiment of the present invention;

FIG. 4B is a cross-sectional view of the transition device of FIG. 4A in accordance with an embodiment of the present invention;

FIG. 5 is a simplified perspective view of a cannulation system including a transition device in accordance with an embodiment of the present invention; and

FIG. 6A is a cross sectional view of a typical drainage hole defined through a cannula; and

FIG. 6B is a cross sectional view of a chamfered drainage hole defined through a cannula in accordance with an embodiment of the present invention.

### DETAILED DESCRIPTION

The disclosure is now described more fully hereinafter with reference to the accompanying drawings, in which some, but not all embodiments are shown. The disclosure may be embodied in many different forms and should not be construed as limited to the embodiments set forth herein. Like numbers refer to like elements throughout.

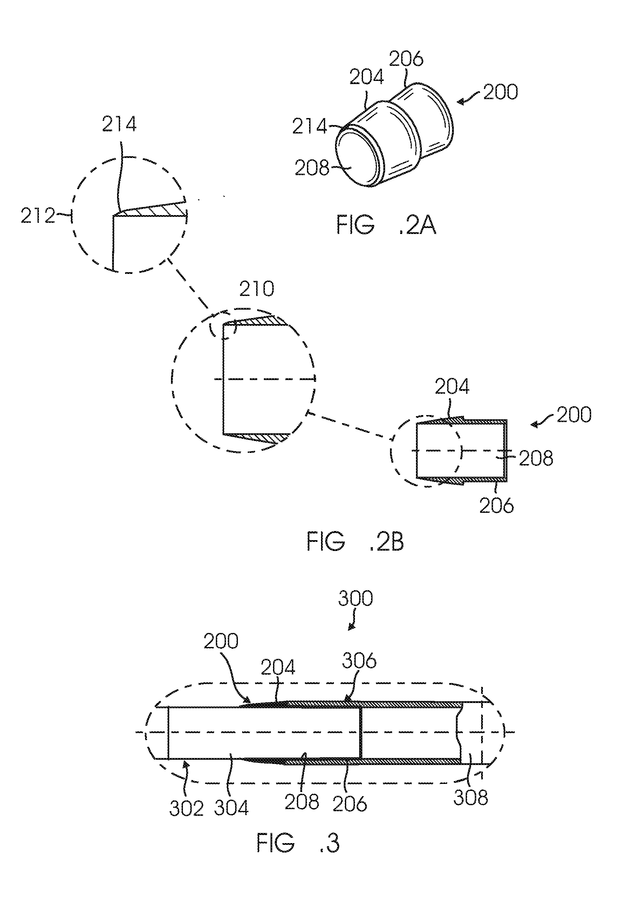

FIG. 2A is a simplified perspective view of a transition device 200 in accordance with an embodiment of the present invention. As shown in FIG. 2A, transition device 200 is a tubular structure formed as a combination of two external sections: a first section 204 having a first geometry, for example, a variable external diameter and a second section 206 having a second geometry, for example, a substantially constant external diameter. In one embodiment, the substantially constant external diameter is formed with a requisite length and diameter to facilitate attachment to cannula tubing, such as by interference fitting second section 206 into the lumen of the cannula tubing. Alternatively, second section 206 may include attachment structures formed thereon or attached thereto, for example, hooks, barbs, laser welding, adhesives or any of a number of possible equivalents well known in the art. Internally, transition device 200 is shown to define an internal lumen 208.

As shown in FIG. 2B, first section 204 is designed such that leading edge 210 of first section 204 includes a tip portion 212 that includes a tip section 214. In one embodiment, tip section 214 is further tapered or otherwise shaped to reduce trauma and to provide for easy insertion, especially through difficult tissue (connective tissue, skin, muscle) such as might be encountered during insertion into the femoral vein.

Transition device 200 may be made of any suitable material that is rigid enough to withstand various loads such as might be encountered during device placement, such as plastic, metal, ceramic, and the like.

FIG. 3 is a simplified cross sectional illustration of a cannulation system 300 in accordance with an embodiment of the present invention. In operation, second section 206 of transition device 200 is positioned within cannula 308.

When second section 206 is positioned within cannula 308, external cannula surface 306 is made to abut against the large end of first section 204. First section 204 creates a gradual and smooth transition between external cannula surface 306 and an object positioned within internal lumen 208 of transition device 200, such as surface 302 of introducer 304. In one embodiment, internal lumen 208 is configured to interface tightly, for example, with an interference fit, with introducer 304.

Tapered tip section 214 (FIG. 2B) on tip portion 212 of transition device 200 further provides and allows for a smooth transition between surface 302 of introducer 304 and cannula surface 306 of cannula 308.

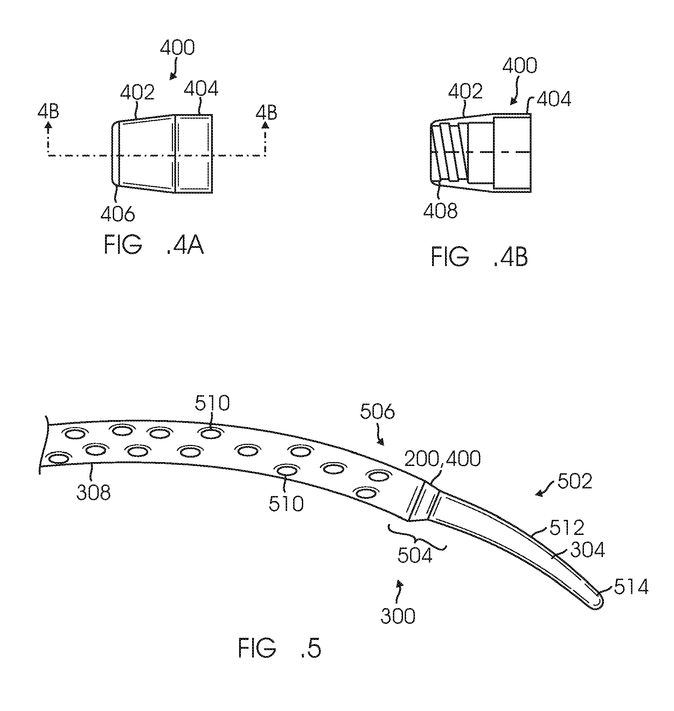

FIGS. 4A and 4B are perspective and cross-sectional views, respectively, of another embodiment of a transition device 400. Externally, transition device 400 is similar to the embodiment of transition device 200 illustrated in FIGS. 2A and 2B. For example, as shown in FIG. 4A, transition device 400 is a tubular structure formed as a combination of a first section 402 having a variable external diameter, a second section 404 having a substantially constant external diameter, and tapered tip section 406 allowing for a smooth transition between the surface of an introducer and a cannula surface.

Internally, as shown in FIG. 4B, transition device 400 includes interior threads 408. Interior threads 408 mate with corresponding threads placed on the exterior surface of an introducer (not shown). Interior threads 408 when mated to the threaded introducer provide a secure lock which helps to maintain the relative position of the cannula and introducer during penetration through difficult tissue, such as may be encountered in a patient with previous scar tissue, or someone with significant obesity.

In one embodiment, interior threads 408 of transition device 400 include a three-start helical square thread with a predetermined height and pitch. Alternatively, interior threads 408 may be a rope thread, which is a thread with a smooth surface relative to typical square or triangular threads.

The threaded transition device 400 acts as a fixation point to control the position of the distal end 502 (FIG. 5) of cannula 308 relative to introducer 304 during cannulation.

As shown in FIG. 5, cannulation system 300 is deflectable at its distal end 502 to permit proper positioning of distal end 502 of cannula 308 including introducer 304 within the femoral vein. However, junction 504 between cannula 308 and introducer 304 having transition device 200 or 400 is no longer prone to exposing an edge during cannulation.

As shown in FIG. 5 introducer 304 includes an elongated tube like structure 512 having a distal end including tip 514.

Elongated tube like structure 512 may be formed of any suitable, material having a sufficiently high durometer to impart substantial rigidity to introducer 304 to facilitate its placement. In one embodiment, tip 514 may be formed of a material, which is substantially softer, i.e., has a substantially lower durometer and molecular weight, than the material of the remainder of tube like structure 512. Using a substantially softer material ensures that tip 514 does not traumatize any tissue with which it engages when introducer 304 is being positioned.

In one embodiment, the high durometer material of tube like structure 512 may have a durometer, for example, in the range of 55 to 75 durometer (Shore D), such as a polyether based polyurethane. In one embodiment, the lower durometer material of tip 514 may have a durometer in the range of 75 to 85 durometer (Shore A), such as aliphatic polyurethane. One particularly effective polyurethane for tube like structure 512 is Pellethane 2363-65D commercially available from Dow Chemical Company of Midland Mich., which has a durometer of 65 (Shore D), while a particularly effective polyurethane for tip 514 is Tecoflex 80A-B20 commercially available from Thermedics, Inc. of Wooburn, Mass., which has a durometer of 80 (Shore A).

In one specific embodiment, introducer 304 is made of polyurethane as the base material. Tube like structure 512 is a blend of HDPE (shore 75D) and LDPE (shore 45D). Tip 514 is made entirely of LDPE. Both tip 514 and tube like structure 512 include BaSO₄ and TiO₂.

Alternatively, other means to impart substantial rigidity to introducer 304 to facilitate its placement other than durometer shift include: the insertion of stiffening members in tube like structure 512, the covering of tube like structure 512 with a stiffening sheath, and chemically treating tip 514 and tube like structure 512.

In one embodiment, the material of tip 514 may include a radiopaque material, such as barium sulfate or the equivalent. The inclusion of the radiopaque material in the material of tip 514, helps to radiographically locate introducer 304 when it is in position, by producing a sharply defined, distinct radiographic image.

Again referring to FIG. 5, in one embodiment of the present invention, cannula 308 includes at least one to a plurality of drainage holes 510 defined between external surface 306 and inner lumen 208 (FIG. 3).

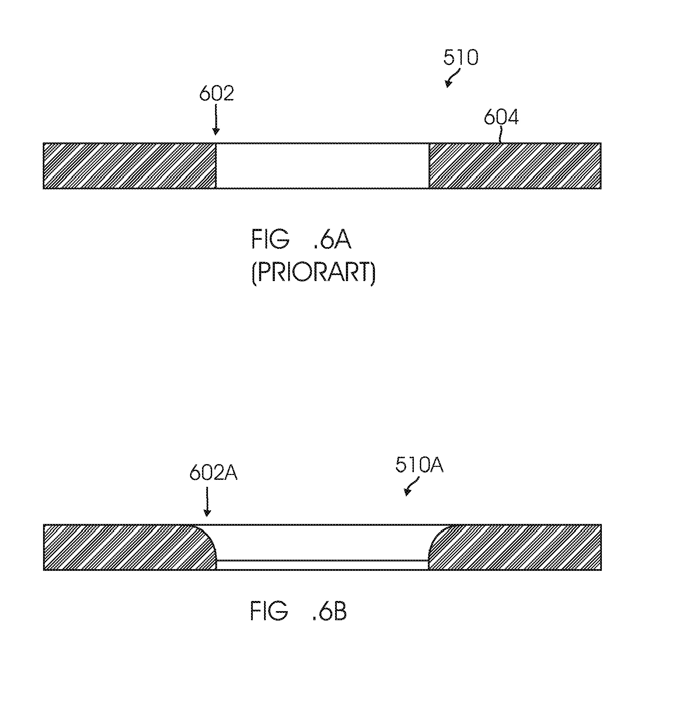

As shown in FIG. 6A, a typical drainage hole 510 includes a square edge 602 and exhibits perceptible roughness to the touch. Generally, hole 510 is punched through the wall 604 of cannula 308 in one stroke. When the punch exits the far side of cannula 308, it may leave a slight-burr or raised edge on external surface 302 (tissue-contacting side of cannula 308) around drainage hole 510. Since cannula 308 is to be inserted through skin, subcutaneous tissue, fat, and connective tissue; then pushed through the femoral vein up to the right atrium of the heart, there is a chance that some of the tissue may be scraped off by the square-edged drainage hole 510. This problem is especially evident during percutaneous insertion, when the surgeon has to push cannula 308 through the most difficult tissue.

As shown in FIG. 6B, in accordance with an embodiment of the present invention, drainage hole 510A may be smoothed, chamfered or given a radius at edge 602A to reduce the possibility of traumatic tissue damage during insertion of cannula 308.

Various methods exist for smoothening edge 602A of hole 510A, including using abrasive material removal, melt profiling, or hot drilling. In an alternative method, the section of cannula 308 which includes drainage holes 510A may be molded from rigid plastic or other similar material to provide a smooth profile.

It is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A cannulation system comprising:
a cannula body having a distal end;
an introducer configured to be insertably received into the distal end of the cannula body; and
a transition device including a first section, a second section and an inner lumen extending therethrough, the first section attached to the distal end of the cannula body, the second section extending outside of the cannula body to receive the introducer into the inner lumen, the second section providing a transition between the cannula body and the introducer such that no gap is opened between the distal end of the cannula body and the introducer as the introducer is flexed relative to the cannula body.

2. The cannulation system of Claim 1, wherein the cannula body comprises at least one drainage hole, the drainage hole including an external edge having a chamfer or radius.

3. The cannulation system of Claim 1, wherein the transition device comprises a threaded section for coupling the transition device to the introducer.

4. The cannulation system of Claim 1, wherein the first section comprises a constant external diameter.

5. The cannulation system of Claim 1, wherein the second section comprises a variable external diameter.

6. The cannulation system of Claim 1, wherein the second section comprises a taper.

7. The cannulation system of Claim 1, wherein the second section comprises a compound geometry of one or more tapers or radii.

8. The cannulation system of Claim 1, wherein the introducer comprises a tube like structure made of a first material with a distal tip made of a second material.

9. The cannulation system of Claim 8, wherein the second material has a durometer value lower than the durometer value of the first material.

10. The cannulation system of Claim 1, wherein the first section includes attachment structures for attaching said transition device to said cannula body.

11. A cannulation system comprising:
a cannula body having a distal end and a first lumen extending therein;
an introducer configured to be insertably received into the distal end of the cannula body; and
a transition device including a first section having a constant external diameter, a second section having a tapered external diameter and an inner lumen extending therein, the first section inserted into the first lumen at the distal end of the cannula body, the second section extending outside of the cannula body and configured to allow the introducer into the first lumen of the cannula body,
the second section providing a transition between the outer surface of the cannula body and the outer surface of the introducer such that no gap is opened at the intersection of the outer surface of the cannula body and the outer surface of the introducer as the introducer is flexed relative to the cannula body.

12. The cannulation system of Claim 11, wherein the cannula body comprises at least one drainage hole, the drainage hole including an external edge having a chamfer, or radius.

13. The cannulation system of Claim 11, wherein the transition device comprises a threaded section for attaching the transition device to the introducer.

14. The cannulation system of Claim 11, wherein the tapered external diameter comprises a compound or simple geometry, such as tapers or radii.

15. The cannulation system of Claim 11, wherein the introducer comprises a tube like structure made of a first material with a distal tip made of a second material.

16. The cannulation system of Claim 15, wherein the second material has a durometer value lower than the durometer value of the first material.

17. The cannulation system of Claim 11, wherein the first section includes attachment structures for attaching said transition device to said cannula body.

18. A cannulation system comprising:
a cannula body having a distal end and a first lumen extending therein;
an introducer configured to be insertably received into the distal end of the cannula body, the introducer including a tube like structure made of a first material with a distal tip made of a second material, the second material having a durometer value lower than the durometer value of the first material; and
a transition device including a first section having a constant external diameter, a second section having a tapered external diameter and an inner lumen extending therein,
the first section inserted into the first lumen at the distal end of the cannula body, the second section extending outside of the cannula body and configured to allow the introducer into the first lumen of the cannula body,
the second section providing a transition between the outer surface of the cannula body and the outer surface of the introducer such that no gap is opened at the intersection of the outer surface of the cannula body and the outer surface of the introducer as the introducer is flexed relative to the cannula body.

19. The cannulation system of Claim 18, wherein the first material has a durometer value in the range of 55 to 75 durometer and the second material has a durometer value in the range of 75 to 85 durometer.

20. The cannulation system of Claim 19, wherein the first section includes attachment structures for attaching said transition device to said cannula body.
